Europäisches Patentamt

(19) European Patent Office

Office européen des brevets

(11) Publication number : **0 574 267 A2**

(12) **EUROPEAN PATENT APPLICATION**

(21) Application number : **93304542.9**

(22) Date of filing : **11.06.93**

(51) Int. Cl.⁵ : **C12Q 1/68**

(30) Priority : **12.06.92 US 898785**

(43) Date of publication of application :
**15.12.93 Bulletin 93/50**

(84) Designated Contracting States :
**AT BE CH DE DK ES FR GB IT LI LU NL SE**

(71) Applicant : **GEN-PROBE INCORPORATED**
**9880 Campus Point Drive**
**San Diego California 92121-1514 (US)**

(72) Inventor : **Ryder, Thomas B.**
**1863 Angeles Glen**
**Escondido, California 92029 (US)**
Inventor : **Kacian, Daniel Louis**
**3911 Tambor Road**
**San Diego, California 92124 (US)**

(74) Representative : **Goldin, Douglas Michael**
**J.A. KEMP & CO. 14, South Square Gray's Inn**
**London WC1R 5LX (GB)**

(54) **Preparation of nucleic acid from blood.**

(57)   The present invention provides a method and kit for preparation of nucleic acid from white blood cells for detection and/or for amplification. The nucleic acid may be prepared by contacting a sample of cells, particularly whole blood, with an appropriate lysing agent under conditions in which red blood cells are lysed but white bloods cells are not. The treated sample may be centrifuged so as to separate desired white blood cells from non-desired red blood cells, and then purifying and lysing said desired white blood cells. The nucleic acid associated with the lysed cells can be detected and/or amplified and the nucleic acid can also be screened for a certain nucleic acid sequence, such as a viral nucleic acid sequence.

EP 0 574 267 A2

The field of the present invention is the preparation of nucleic acid for study, research and investigation.

It is common to require nucleic acid to be isolated and purified (i.e., prepared) from various tissues in order to detect the presence of a particular nucleic acid -- for example, the presence of HIV-1 DNA or RNA in a blood cell of a human. For this purpose, the nucleic acid is generally extracted after extensive purification of appropriate blood cells, lysis of these cells, and purification of the released nucleic acids to remove substances that might inhibit later analytical procedures. In particular, it is important to produce sufficient nucleic acid of a quality and purity to allow its amplification.

Two common methods which allow amplification of a specified sequence of nucleic acid (e.g., deoxyribonucleic acid (DNA) or ribonucleic acid (RNA)) are one termed the "polymerase chain reaction" (where two primers are used to synthesize nucleic acid lying between the regions where the primers hybridize), and one which uses RNAse H, reverse transcriptase and RNA polymerase. These methods are described respectively by Mullis et al., U.S. Patent 4,683,202 and by Kacian et al., PCT/US90/03907 (published as WO91/01384) both hereby incorporated by reference herein.

All references, including publications, patents and patent applications are incorporated herein in their entirety by this reference.

## SUMMARY OF THE INVENTION

The present invention is directed to methods and kits for the preparation of nucleic acid, particularly for isolation of sufficient quantity and quality of DNA or RNA from white blood cells, for amplification of that nucleic acid. Such amplified nucleic acid may then be used for various purposes, including screening the nucleic acid for the presence of viral nucleic acid sequences using a probe which is complementary to a selected nucleic acid sequence of the virus. It is also useful for detection of genetic anomalies or defects in the nucleic acid. Accordingly, the methods and kits are designed to allow rapid and easy preparation of nucleic acid from preparations containing both red blood cells and white blood cells (such as whole blood) without the need for extensive purification procedures.

Thus, in a first aspect, the present invention features a method for isolating nucleic acid from white blood cells in an amount sufficient for detection of the nucleic acid. A sample containing cells including red blood cells and white blood cells (e.g., whole blood) is contacted with an agent which under certain conditions is capable of differentially lysing the cells, that is, lysing red blood cells but not white blood cells. By "nucleic acid" is meant DNA or RNA or their equivalents. The phrases "red blood cell" and "white blood cell" are used in their art-recognized manner. By "lysed" is meant that the red blood cells are disrupted and the cells and their contents are rendered readily separable from the intact white blood cells, for example, by a short centrifugation forming a pellet of the intact white blood cells. With reference to white blood cells, the term "intact" indicates that the cell continues to retain its nucleic acid in a form suitable for amplification. Thus, by "intact" is meant that the white blood cells can be readily separated from lysed red blood cells and that the nucleic acid to be detected remains in amplifiable form. The term is not related to viability of a white blood cell. Not all red blood cells are completely lysed by the differentially lysing agent, and not all white blood cells remain intact after contacting with the lysing agent. Thus, by "lysing red blood cells but not white blood cells" is meant that generally at least about 80%, preferably at least about 85% of the originally intact red blood cells are lysed and at least about 30% of the originally intact white blood cells remain intact; most preferably, about 95% of the originally intact red blood cells are lysed and at least about 50% of the originally intact white blood cells remain intact.

The method may be used to obtain nucleic acid from whole blood, either undiluted or diluted up to about 10-fold; preferably diluted less than about 5-fold; and more preferably less than about 3-fold. Blood may be obtained from a human or from another source.

The lysing agent employed in the presently claimed methods and kits may be a surfactant, such as a saponin, detergent or other emulsifying agent. Preferably, the surfactant is a saponin. Other preferred agents include the cationic surfactants cetrimide and cetyl trimethylammonium bromide and detergents with similar structures. Other lysing agents useful in the present invention include toxins, such as toxic fractions from Gymnodium, macrolides and various bacterial hemolysins.

When DNA is desired to be isolated, the integrity of the cell membrane of the white blood cells harvested will not be as critical as when RNA is the nucleic acid sought to be isolated. Thus, the selection of lysing agent will depend, at least to a certain extent, on the type of nucleic acid sought to be detected and/or amplified.

In another aspect, the present invention features a method for preparing nucleic acid for amplification comprising the steps of contacting a white blood cell- and red blood cell-containing population of cells with a lysing agent under conditions in which red blood cells but not white blood cells are lysed, thereby leaving white blood cells intact; purifying said intact white blood cells; and contacting said intact white blood cells with a lysing agent able to lyse said white blood cells to release the nucleic acid sought to be detected and/or amplified.

Purification of the white blood cells may be accomplished by any suitable method, for example, by centrifugation at about 7500 rpm for 5 min to pellet the white blood cells.

Lysing of the purified white blood cells may be performed by any desired method, including the use of a strong alkali (such as $(CH_3)_3NOH$ potassium hydroxide, sodium hydroxide or lithium hydroxide), an enzymatic agent, a surfactant (such as a saponin), osmotic shock, a chaotropic agent or sonic disruption. In a preferred embodiment, the white blood cells are lysed with alkali. Such a lysis also denatures any double stranded nucleic acid from the cells, thereby facilitating amplification.

In yet another aspect, the present invention features a method for amplifying nucleic acid comprising the steps of contacting a white blood cell- and red blood cell-containing population of cells with a lysing agent under conditions in which red blood cells but not white blood cells are lysed, thereby leaving white blood cells intact; purifying said intact white blood cells; contacting said intact white blood cells with a lysing agent able to lyse said white blood cells; and amplifying said nucleic acid.

Amplification of the nucleic acid may be accomplished by any desired method, including the Mullis method and the Kacian method referenced above.

In preferred embodiments, the method includes adding a complexing agent that complexes with, and effectively removes, ferric ions ($Fe^{+++}$) from the solution resulting from lysis of the white blood cells before amplifying the nucleic acid.

Once the nucleic acid has been amplified, methods are known in the art for screening the amplified nucleic acid using known nucleic acid hybridization and detection techniques. For example, by using a nucleic acid probe complementary to a target sequence (which preferably includes the sequence targeted for amplification), hybridizing the probe to the target nucleic acid sequence, and detecting the complex of probe and target by well known methods, such as Southern or northern blots or by the homogeneous solution phase procedure ("HPA") described in Arnold et al, 35 Clin. Chem. 1588 (1989), and PCT U.S. 88/02746, all of which are hereby incorporated by reference. By the use of such a probe detection method, it can be determined whether viral nucleic acid, such as that from HIV or hepatitis B virus (HBV), is associated with the nucleic acid present in the sample. Such a screening may also target any other sort of nucleic acid sequence, such as a genetic anomaly or defect. The use of such amplification procedures allows even a single copy of the targeted nucleic acid in the portion of the sample recovered after centrifugation to be detected using the present invention.

In still another aspect of the present invention, a kit is provided, including apparatus, media and agents necessary to conduct the above described inventive method. For example, the kit may include a supply of a lysing agent sufficient to lyse the cells from the sample, and a supply of the amplification materials sufficient to amplify the nucleic acid from the cells. Such a kit may also include a ferric ion and/or calcium ion complexing agent, a supply of the probe for a desired nucleic acid target, or a known genetic anomaly and a device for the collection and storage of whole blood.

Other features and advantages of the invention will be apparent from the following description of the preferred embodiments, and from the claims.

BRIEF DESCRIPTION OF THE DRAWINGS

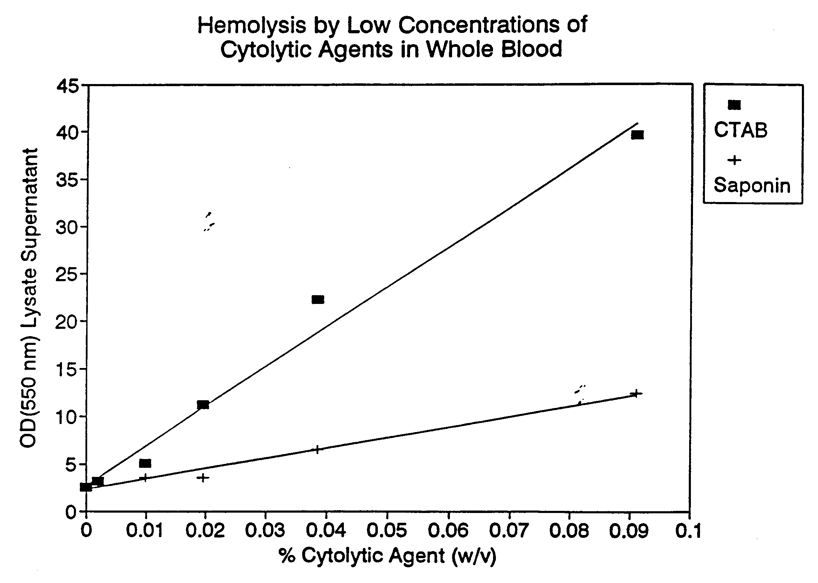

Figure 1 shows the optical densities observed, corrected for dilution, of samples after treatment with CTAB or saponin. The amount of hemolysis is shown by optical density at 550-nm of the lysate supernatant. The concentration of detergent is given as percent weight/volume. The values for CTAB are shown as filled in squares. The values for saponin are shown as + symbols.

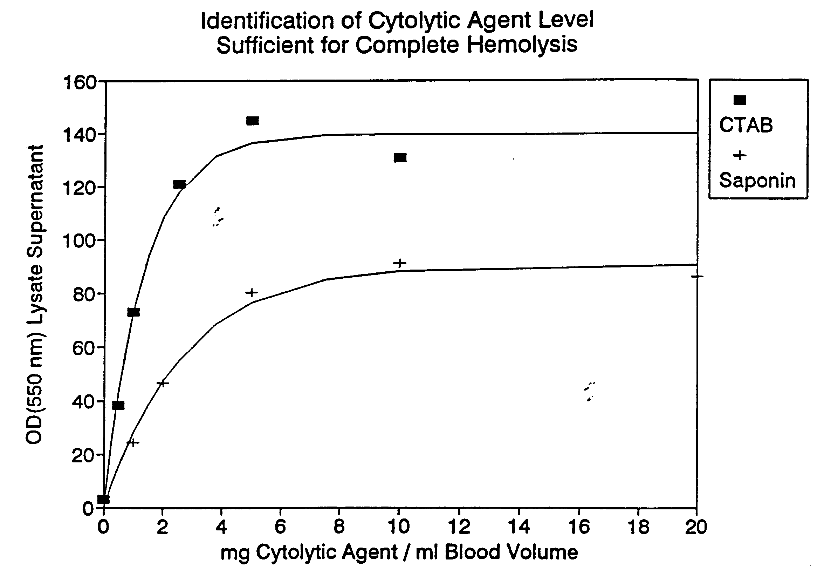

Figure 2 shows the amount of hemoglobin released per amount of detergent added. The amount of hemoglobin released is shown as optical density at 550 nm of the lysate supernatant. The amount of detergent added is shown as mg detergent/ml blood volume. Values for CTAB are shown as filled in squares. Values for saponin are shown as + symbols.

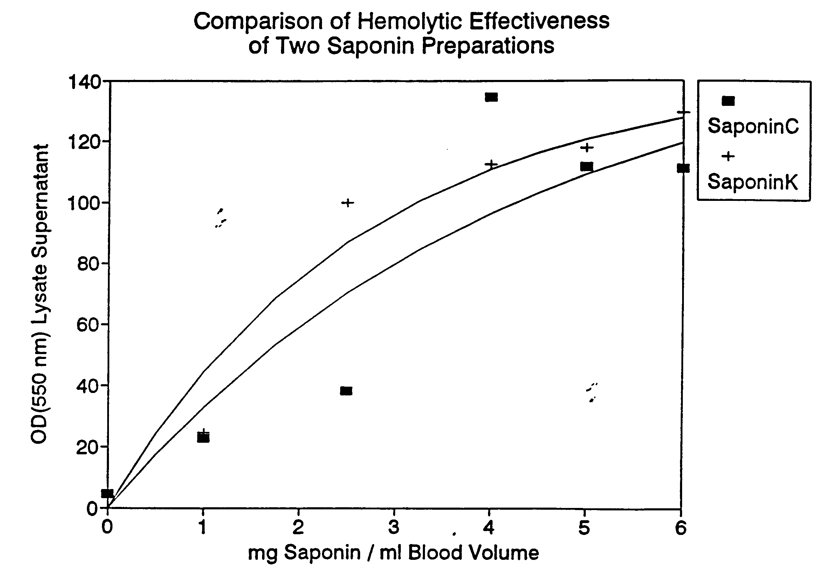

Figures 3 shows a comparison of hemolytic effectiveness of two saponin preparations: saponinC (filled in squares) and saponinK (+ symbols). Hemolytic effectiveness is shown as optical density of the lysate supernatant at 550 nm. Amount of saponin preparation is shown as mg saponin/ml blood volume.

DESCRIPTION OF THE PREFERRED EMBODIMENTS

The claimed method features a process for preparing nucleic acid for amplification, preferably from a population of cells containing both red blood cells and white blood cells, such as whole blood, and a combination of apparatus, media and agents to effect such a method. The various steps, apparatus, media and agents are discussed generally above, and examples are now provided.

## Obtaining A Sample

A sample containing red blood cells and white blood cells may be provided from any suitable source, such as from whole blood. Preferably, the sample comprises anticoagulant treated whole blood, which may be obtained from any animal including a human being. The blood may be pretreated if desired, e.g., to remove fibrinogen. Other blood fractions or any other fluids containing white blood cells can be used.

The blood, or other source of a sample of cells, may be obtained using any available method, including those known in the art. The sample can be obtained and stored, and even subjected to centrifugation, in the same device. If the sample is blood, it may be desirable to treat the sample with an anti-coagulant, such as EDTA or heparin, so that the blood does not coagulate prior to performance of the rest of the method, or during use of the rest of the materials and apparatus in the kit.

## Pretreatment

The blood may be the pretreated if desired, for example, to remove fibrinogen. Other useful pretreatments include preparing a white blood cell-enriched supernatant using a carbohydrate polymer, e.g. dextran, hydroxyethyl starch, hydroxymethyl cellulose or other red blood cell-agglutinating agents by methods well known in the field. Of course, if such pretreatment is used, the differential lysis methods described herein must be optimized for pretreatment samples.

## Differential Lysing

Procedures for differential lysing are described in Examples 1-7 herein. Various agents can be used to perform the differential lysing. A review of various types of lysing agents is found in Thelestam & Möllby, 557 Biochim. Biophys. Acta 156 (1979). For example, saponins, which are plant glycosides having the property of forming a soapy lather when shaken with water, can be used. Mahato, et al., 21 Phytochemistry, 959 (1982). Saponins, in general, are very powerful emulsifiers and are able to form a complex with cholesterol. Saponins are classified as steroid or triterpenoid saponins, depending on the nature of the aglycone. For a general review of saponins, see, e.g., Vogel, 11 Planta Med., 362 (1963); Tschesche and Wulff, 12 Planta Med, 272 (1964); Kochetkov and Khorlin, 16 Arzneim, Forsch 101 (1966); Kawasaki, 16 Sogo Rinsyo 1053 (1967); Tschesche, 25 Kagakuno Ryoiki 571 (1971); Kawasaki, 11 Method. Chim. 87 (178); and Tschesche and Wulff, 30 Fortschritee der Chemie Organischer Naturstoffe 461 (1973), the aglycone of a steroid saponin usually being a spirostanol or derivative thereof. See, Elks, 11E Rodd's Chemistry of Carbon Compounds, 1 (1971); and Elks, 2D Rodd's Chemistrv of Carbon Compounds, 205 (1974). For a discussion of triterpenoid saponins, see, Basu and Rastogi, 6 Phytochemistry 1249 (1967); Agarwai and Rastogi, 13 Phytochemistry 2623 (1974); and Chandel and Rastogi, 19 Phytochemistry 1889 (1980). A third group of saponins, which contain nitrogen analogs of steroid sapongenins as aglycones, are called basic steroid saponins, see Schreiber, 10 Alkaloids 1 (1968); Roddick, 13 Phytochemistry, 9 (1974); Herbert, 5 Alkaloids, 256 (1975); and Harrison, 6 Alkaloids, 285 (1976). Preferred saponins include preparations available from Calbiochem (La Jolla, CA) and Kodak (Rochester, NY).

Other detergents may also used as lysing agents. See, e.g., Helenios and Simons, 415 Biochim. Biophys. Acta 29 (1975); Bonsall and Hunt, 249 Biochim. Biophys. Acta 266 (1971). Cationic surfactants are particularly useful as lysing agents. See generally Isomaa, et al., 44 Acta Pharmacol. et. Toxicol. 36 (1979). For example, cetyl trimethylammonium bromide (CTAB) may act by filling up binding sites and interacting electrostatically with negative charges on the cell surface to perhaps induce changes in the surface topography or membrane ultrastructure, thereby influencing electrophoretic mobility, or by inducing rearrangements of negatively charged particles at the cell surface. For example, it has been reported that lysolecithin, a surface-active echnocytogenic substance, reduces the binding of ferritin particles to the erythrocyte surface. Marikovsky et al., 98 Exp. Cell Res. 313 (1976). See also Nakagawaia & Nathan, 56 J. Immunoloqy Methods 261 (1983). The detergent cetrimide can also be used as an agent for differential lysis. See Gagon et al., 46 Am. J. Clin. Pathol. 684 (1966); D'Angelo and Lacombe, 32 Bull. Res. M. Technol. 196 (1962). The term cetrimide is sometimes used to describe CTAB and structurally similar detergents, such as dodecyl trimethylammonium bromide, tetradecyl trimethylammonium bromide, and cetyl dimethylethylammonium bromide.

Other agents useful in the presently claimed methods and kits include various short-chain phospholipids, as described in Mashino et al., 94 J. Biochem 821 (1983). For example, phosphatidylcholines with chain lengths from 8 to 12 carbon atoms have been reported to lyse erythrocytes. Id.

Other agents, such as certain snake venoms, also have hemolytic activity and thus, are suitable for use as differential lysing agents provided suitable conditions are used. See Gul et al., 12 Toxicon 311 (1974), which reports data on hemolysis of red blood cells by various snake venoms, and which reports a correlation between hemolysis and phospholipase A activity. Other lysing agents include toxins, e.g., toxic fractions from a Florida red tide organism, Gymnodinium breve. Kim et al., 12 Toxicon 439 (1974). Macrolides and thiol-activated hemolysins are also suitable for use as lysing agents.

As noted above, agents which interact with red blood cells to a greater extent than with white blood cells, thereby causing differential lysis, are useful in the present invention. Methods for determining whether a particular agent will be useful as a differential cytolytic agent are described herein.

Some aspects of the rate and extent of cytolysis by agents, including detergents, such as a saponin or cetyl trimethylammonium bromide (CTAB), can be described in terms of the concentration of detergent molecules, the concentration of binding sites available on all cells present and the relative affinity of different detergents for different sites (Bonsall et al., 249 Biochim. Biophys. Acta 266 (1971). Cytolysis seems to correlate with binding events in excess of a threshold number of sites on a cell. Cytolytic agents with relatively more activity against certain cell types might have differential affinity, binding kinetics or cytolytic threshold sites for that cell type. In some cases a membrane might be nearly devoid of binding sites, for example a principal binding determinant for many saponins is the presence of cholesterol and membranes which are low in sterol content are typically resistant to saponin lysis. However many agents which are particularly effective at hemolyzing red blood cells also have some activity against white blood cells. For example, our investigations showed that to effectively lyse all red blood cells present in whole blood, or in a mixture in which a significant fraction of the total volume is contributed by whole blood, it was often necessary to use levels of cytolytic agents in a concentration which caused lysis of the white blood cells also. Furthermore the composition of blood from different individuals can vary significantly; for example, the level of red blood cells present can vary over at least a two-fold range. Therefore it was unexpected that conditions and concentrations can be identified for any particular cytolytic agent under which these goals can be achieved consistently in mixtures containing a significant partial volume contributed by blood. Described herein are methods for identification of suitable agents and effective conditions for differential lysis.

Purification of White Blood Cells

The white blood cells are then purified, for example, by centrifugation such that the desired intact white blood cells effectively move to form a pellet of isolated cells separate from lysed red blood cells. For instance, the mixture is centrifuged at about 7,500 rpm for about 5 minutes.

In some cases it is desirable to perform an additional step to wash and further purify the white blood cells recovered in the pellet of such a centrifugation procedure. Because the solution used to wash the white blood cells can also include a hemolytic agent, it is not necessary to completely lyse all red blood cells in the initial treatment but the amount lysed should be sufficient to facilitate further handling of the cells recovered and to yield white blood cells consistently of a desired degree of purity after the purification step.

If it is desirable to avoid the additional handling required for a wash step, it is possible to use the results of the type of experiment described in Example 2 to suggest a range of cytolytic agent levels which are likely to yield complete hemolysis in virtually every sample. To utilize this strategy it is desirable to identify conditions under which the excess activity of the cytolytic agent does not lyse the desirable white blood cells. Tests for the number and types of white blood cells recovered are straightforward and are described in Examples 4-6 herein.

Lysis of White Blood Cells

After removing the isolated desired white blood cells, the cells are lysed to release the nucleic acid from, or associated with, the cells. This means any nucleic acid either attached to or found within or on the desired cells.

The cells may be lysed by any desired method, including, for example, the use of a strong alkali, use of an enzymatic agent, use of a detergent, use of osmotic shock, use of chaotropic concentrations of solutes, or use of sonic disruption. In a preferred embodiment, the white blood cells may be lysed by use of potassium hydroxide. This alkali, or an equivalent alkali, e.g., NaOH or LiOH, is particularly useful since it also inactivates cellular nucleases and thus prevents inhibition of later enzymatic reactions using the alkali-induced lysate, and may also denature double stranded nucleic acid.

Amplification Of Nucleic Acid

The nucleic acid may now be amplified using any method known in the art, such as a polymerase chain reaction procedure, or a procedure described by Kacian, supra, using RNAse H, reverse transcriptase and RNA polymerase. Preferably, the amplification will target the same sequence of nucleic acid that will be the target of the probe, discussed below.

## Screening For Desired Nucleic Acid

The amplified nucleic acid is now ready to be screened for a desired sequence of nucleic acid using any procedure known in the art or other related procedures, such as hybridization using a strand of DNA or RNA complementary to the desired nucleic acid sequence, which complementary strand is known as a "probe." The probe may be hybridized to the nucleic acid and detected using any known detection technique, such as traditional Southern or Northern blotting techniques, or the homogeneous protection assay ("HPA") described by Arnold et al., supra. In a preferred embodiment, the probe will detect the nucleic acid associated with an HIV virus.

## Using the White Blood Cell Lysate in Amplification Reactions

The nucleic acid present in the white blood cell lysate, while fragmented and denatured compared to its original structure within the cells, is still a suitable reactant for a variety of chemical and biochemical reactions, including as a template for nucleic acid target amplification. However, it is well known that biologically-derived samples frequently contain components that are inhibitory to in vitro biochemical reactions, including enzyme reactions. See, e.g., R. deFranchis, et al. 16 Nucleic Acids Research 10355 (1988).

Thus, in one embodiment of the claimed invention, a complexing agent may be added to the white blood cell lysate. The complexing agent is preferably selected so that it complexes with ferric ions ($Fe^{+++}$), but does not complex with zinc ions ($Zn^{++}$), manganese ions ($Mn^{++}$) or magnesium ions ($Mg^{++}$), as these ions are beneficial and tend to facilitate enzymatic reactions. Such an ager is the chelating agent deferoxamine (also known as desferrioxamine and desferrioxamine B).

## Kit For Effecting The Above Methods

A simple kit for performing the above method may be prepared from readily available materials and reagents.

## EXAMPLES

To assist in understanding the invention, the results of a series of experiments follow. The following Examples relating to the present invention should not, of course, be construed as specifically limiting the invention, and such variations of the invention, now known or later developed, which would be within the purview of one skilled in this art are to be considered to fall within the scope of this invention as hereinafter claimed.

## Example 1: Determination of Efficacy of Low Concentrations of Cytolytic Agents in Whole Blood

To determine detergent concentration effective for lysing red blood cells when added directly to whole blood, the following titration was performed.

1) Whole blood was collected into a tube containing EDTA as an anti-coagulant. 500 µl aliquots of blood were dispensed into several centrifuge tubes.

2) 0, 1, 5, 10, 20 or 50 µl of a 1% (w/v) solution of CTAB was added to one series of blood aliquots. Another series of blood aliquots received the same volumes of a 1% (w/v) saponin solution. The tubes were inverted to mix and the tubes were incubated 5 min at room temperature.

3) The tubes were then centrifuged at 7500 RPM for 5 min to pellet unlysed cells. 250 µl supernatant was removed from each tube.

4) The amount of hemoglobin released into the supernatant was determined by measuring the absorbance of light at 550 nm. Samples were diluted as necessary so that the optical density of each sample was within the linear response range of the spectrophotometer.

Fig. 1 shows the optical densities observed, corrected for dilution. It appears that CTAB may be a more effective hemolytic agent in this concentration range than saponin, but the extent of hemolysis was incomplete for both; the amount of hemoglobin released was still increasing linearly with the detergent level in both cases.

## Example 2: Identification of Conditions Effecting "Complete" Hemolysis of Red Blood Cells

The following experiment was performed using a broader concentration range of the hemolytic agent being tested than was used in Example 1 since it was evident that those conditions did not cause complete hemolysis.

1) Stock solutions comprising 10% (w/v) saponin in 0.15 M NaCl and 5% CTAB in 0.15 M NaCl were prepared.

2) Whole blood was collected into a tube containing EDTA as an anti-coagulant. 500 μl aliquots of blood were dispensed into several centrifuge tubes.

3) 0, 5, 10, 25, 50 or 100 μl of the CTAB solution was added to one series of blood aliquots. Another series of blood aliquots received the same volumes of the saponin solution. The tubes were inverted to mix and the tubes were incubated 5 min at room temperature.

4) The tubes were centrifuged at 7500 RPM for 5 min to pellet unlysed cells. 100 μl supernatant was removed from each tube and diluted into water.

5) The amount of hemoglobin released into the supernatant was determined by measuring the absorbance of light at 550 nm. Additional dilutions were made as necessary so that the optical density of each sample was within the linear response range of the spectrophotometer.

Fig. 2 shows the amount of hemoglobin released per amount of detergent added. CTAB again appeared to have more hemolytic activity per mass added but also yielded supernatants of somewhat higher optical density. Both agents effected complete hemolysis in the concentration range tested as shown by the broad plateau of maximum OD(550). For example, under the conditions described, 2.5 mg saponin is sufficient to lyse >85% of the red blood cells in 0.5 ml blood, and likewise, 1.25 mg CTAB lysed >85% of the red blood cells in 0.5 ml blood. If such a secondary hemolytic wash is to be employed, it may be desirable to choose levels of the primary hemolytic agent near the edge of the plateau, as shown here. This would minimize the likelihood of white blood cell lysis by excess free activity of the cytolytic agent.

## Example 3: Determination of Satisfactory Washing Agents

The white blood cell pellet obtained from the primary hemolysis procedures described in Examples 1 and 2 can be resuspended and diluted in a solution with suitable properties (e.g., isotonic to desirable cells) and then centrifuged or filtered to reconcentrate and recover the white blood cells. Such a wash is frequently desirable to dilute and remove soluble components remaining in the residue of the supernatant and in the interstitial volume. Such a wash can also be used to complete the hemolysis of red blood cells which did not lyse in the initial treatment.

The experiments described in this example investigated the stability of purified white blood cells to dilution and incubation in three hemolytic agents, including CTAB and saponin at concentrations similar to those shown in Examples 1 and 2 to be effective at lysing most red blood cells in whole blood. $NH_4Cl$ (or some other ammonium ions and salts) can be used for differential cell lysis. For example, a sample containing red blood cells may be diluted into a significant excess of buffered $NH_4Cl$ solution of near-physiological osmolarity. In this example, mononuclear white blood cells (MNC) prepared by density gradient centrifugation were used to particularly investigate the stability of the white blood cells subset which includes the target cells for HIV infection, but total white blood cells or other subsets prepared by other methods can also be used.

1) A suspension containing MNC was prepared by fractionating whole blood by standard methods employing Ficoll-hypaque density gradient centrifugation.

2) Harvested MNC were diluted in saline to wash; the suspension was aliquoted into several individual centrifuge tubes (equivalent to about 0.3 ml original blood volume per tube) and centrifuged to pellet MNC.

3) Pellets were suspended in 0.1 ml of either: 0.25% CTAB in 0.15 M NaCl, 0.5% saponin in 0.15 M NaCl, or 0.14 M $NH_4Cl$, 0.015 M Tris-Cl, pH 7.6 (1X $NH_4Cl$). The tubes were incubated at room temperature; at 0, 10 30 and 60 min, a 10 μl aliquot was removed for cell counts and microscopic examination in hemocytometer. Cell counts in the table below were normalized to the original corresponding blood volumes.

TABLE 1

| Wash | t= 0 min | 10 min | 30 min | 60 min |
|---|---|---|---|---|
| NH$_4$Cl | 1.95 X 10$^6$ | 1.5 X 10$^6$ | 2.2 X 10$^6$ | - |
| | Good cell morphology | Good cell morphology, slight distension | Cells becoming distended | Most objects are nuclei |
| CTAB | 3.2 X 10$^6$ | - | - | - |
| | Cells are in clumps with debris | Large clumps of debris | Large clumps of debris | Clumps of debris. Perhaps some cells |
| Saponin | 1.9 X 10$^6$ | 2.7 X 10$^6$ | - | - |
| | Most cells have significant membrane distensions | Cell show distention | Most object probably nuclei | Debris and membrane vesicles |

In most samples where the cells had been exposed to CTAB, the cells (and debris) tended to clump, perhaps because of a tendency of CTAB to aggregate polyanions, resulting in higher than expected cell counts in CTAB-treated samples. These results described in Table 1 show that absolute concentrations of detergents necessary for nearly complete red blood cell lysis in whole blood, are excessively cytolytic for purified white blood cells. More dilute solutions of the detergents, especially saponin, which would yield improved white blood cell stability and conditions giving acceptable extents of residual red blood cell hemolysis can be identified by similar techniques. However the stability in 1X NH$_4$Cl was the best of the three under the conditions tested and would be adequate for many practical applications.

In some of these samples, it appeared that, although the cells had lysed, the nuclei remained intact. For many applications using the chromosomal DNA as a source of nucleic acid, this might also be an acceptable or even desirable processing product. Centrifugation conditions sufficient to recover intact nuclei are known and can be validated with routine experimentation. Many mild (especially non-ionic) detergents are known which lyse cells readily but do not denature nuclei.

Example 4: Processing Yield and Cell Integrity

As another measure of cellular integrity through the processing steps, the ribosomal RNA (rRNA) content of the cells recovered after processing, (as described below) was measured by quantitative solution hybridization. This analysis is useful to determine conditions under which intracellular RNA target analytes are preserved.

1) Whole blood was obtained using EDTA as an anti-coagulant, and 3 ml was dispensed into each of several centrifuge tubes. 150 μl of 10% saponin in 0.15 M NaCl was added to one; 150 μl of 5% CTAB in 0.15 M NaCl was added to another; and 300 μl of 1.5 M NH$_4$Cl was added to a third. The tubes were inverted to mix and incubated 5 min at room temperature.

2) The tubes were centrifuged for 3 min at 8000 RPM, and the supernatant decanted.

3) Pellets were resuspended with 1.5 ml 1X NH$_4$Cl. 1/3 of each resuspended volume was dispensed to each of three centrifuge tubes, and centrifuged for 1 min at 8000 RPM.

4) Each pellet was resuspended in 200 μl balanced saline solution. A sample of the resuspended cells was examined by hemocytometer counting and another sample was mixed with 2% sodium lauryl sulfate to lyse the cells for hybridization analysis.

5) Solution hybridization reactions using these lysates were performed according to the method described in Kohne, U.S. Patent No. 4,851,330, entitled "Method for Detection, Identification and Quantitation of Non-Viral Organisms," naming David E. Kohne as inventor, which is incorporated herein by the reference, i.e., using a probe, labeled with $^{125}$I, which is complementary to a rRNA sequence strongly conserved in cellular organisms. rRNA-probe hybrids were separated from unhybridized probe using hydroxylapatite, as described by Kohne, supra, by contacting the products of the hybridization reaction with hydroxylapatite in the presence of 0.12 M sodium phosphate pH 6.8. After centrifugation, the hydroxylapatite-bound sediment was washed at 65° in the same buffer.

The whole blood sample which received NH$_4$Cl as the intended, primary lytic agent, showed virtually no evidence of hemolysis after the first centrifugation. Thus, NH$_4$Cl appears not to be as effective as saponin and CTAB as a primary lytic agent when most of the mixture volume is derived from the whole blood, perhaps because the cells are stabilized by the normal ions contributed from the plasma. (The packed red blood cell mass resulting from the initial centrifugation showed nearly complete hemolysis after it was resuspended in the 1X NH$_4$Cl washing solution.)

The cell counts and the hybridization signals in Table 2 are normalized to correspond to the yield of 1 ml original blood volume. The hybridization signals represent the average cpm obtained from hybridization reactions with lysates of three replicate white blood cell pellets for each treatment or from three replicate control samples.

TABLE 2

| Sample | Cell/ml | cpm |
|---|---|---|
| Initial Saponin Lysis | 8 X 10$^6$ | 43172 |
| Initial NH$_4$Cl Lysis | 8.8 X 10$^6$ | 22104 |
| Initial CTAB Lysis | 9.6 X 10$^6$ | 9004 |
| Hybridization Pos Ctl | N/A | 53523 |
| Hydridization Neg Ctl | N/A | 80 |

Quantitative characterization of the probe used in this experiment showed that hybridization to the rRNA content of 1 X 10$^6$ white blood cells would have been expected to give about 10,000 cpm in this experiment (assuming 1.25 pg rRNA/WBC; see Soren and Biberfeld, 79 Exp. Cell Res., 359 (1973). The rRNA content of the saponin-lysed cells was within about 2-fold of the estimated value expected from the cell counts, suggesting excellent cell integrity from-this processing. The relative values of the signals from the different processing strategies suggest that these conditions for CTAB hemolysis may not be preferable when the goals of the processing include preserving the stability of RNA molecules.

Example 5: Cell Type Recovery

In addition to total cell yield (see Example 4, supra) , it is also useful to characterize the cells recovered to determine if the desired target cell type(s) are recovered with adequate stability. In this example, total cell counts and differential cell-type ratios were determined for blood processed after collection into three different anti-coagulants.

Table 3 shows the normal concentrations of white blood cell types in peripheral blood.

TABLE 3

| Cell Type | Cells/$\mu$l | % Total |
|---|---|---|
| Neutrophil | 3650 | 54.0 |
| Lymphocyte | 2500 | 37.0 |
| Monocyte | 430 | 6.4 |
| Eosinophil | 150 | 2.2 |
| Basophil | 30 | 0.44 |
| **Total** | **6760** | |

The procedure used to characterize cells recovered is as follows:

1) Whole blood was drawn from 2 individuals into evacuated collection tubes containing either EDTA, heparin or sodium citrate as anti-coagulant. An additional sample was collected without anti-coagulant to make serum for the Wright staining procedure (described below).

2) 5 ml of each type of anti-coagulant-treated blood was transferred into a centrifuge tube, and 250 $\mu$l 10%

saponin was added. Samples were inverted to mix, and incubated at room temperature for 5 min. White blood cells were pelleted by centrifugation.

3) Pellets were resuspended in 2.5 ml 1X $NH_4Cl$. 0.5 ml aliquots of each suspension were transferred to respective series of centrifuge tubes, and cells were pelleted by centrifugation.

4) One pellet corresponding to each anticoagulant treatment was resuspended in 100 µl balanced saline solution for hemocytometer counts. One pellet of each type was resuspended in 100 µl serum. The serum-resuspended samples were smeared on glass slides and stained by immersing the dried smear in Wright's stain (Fisher Diagnostics, Pittsburg, PA) for 3 min followed by rinsing with water. A cell type was assigned, based on characteristic staining behavior, to at least 200 cells per slide.

Table 4 below shows the results of these experiments. The total counts (cells/ml) have been normalized to correspond to 1 ml original blood volume. The values for the cell types are the percent of all cells observed from that sample which correspond to the labeled category.

TABLE 4

| Do-nor | Anti-Coagulant | Cells ml | Baso-phils | Eosin-ophils | Neutro-phils | Lympho-cytes | Mono-cytes |
|---|---|---|---|---|---|---|---|
| 1 | Heparin | $3.7 \times 10^6$ | <0.5 | 3 | 60.5 | 23 | 13.5 |
| | EDTA | $2.0 \times 10^6$ | <0.5 | 2.5 | 64.5 | 24 | 9 |
| | Citrate | $3.2 \times 10^6$ | 0.5 | 3 | 53 | 27.5 | 16.5 |
| 2 | Heparin | $2.4 \times 10^6$ | 1 | 5.5 | 68.5 | 14 | 11 |
| | EDTA | $3.8 \times 10^6$ | <0.5 | 2.5 | 42 | 44 | 11.5 |
| | Citrate | $3.6 \times 10^6$ | <0.5 | 5.5 | 49 | 34 | 11.5 |

These results show good yields and indicate normal, and largely consistent, differential cell yields for all three anticoagulants tested.

Example 6: Comparison of Saponin from Two Different Sources

As noted above, the term saponin refers to a large category of biologically derived surfactants with similar, operationally defined properties. Mahato et al., supra. Many of them share subsets of structural features, but across the whole category a wide range of properties might be expected for any one activity (e.g. hemolytic efficacy). Furthermore most commercial preparations are crude mixtures of molecules and there are a number of different plants which are commonly used as sources. Thus for successful practical application of methods employing saponins, it is valuable to have a series of analyses such as described herein to judge the quality and suitability for a particular purpose. The experiments above were all done with the same lot of saponin (from Calbiochem, La Jolla, California, called saponinC in the description below). This example shows a comparison to the properties of another saponin preparation (from Kodak, Rochester, New York, called saponinK below).

1) Solutions of each saponin which were 10% (w/v) of the respective saponin in 0.15 M NaCl were prepared.

2) Whole blood was drawn into a tube containing a suitable anti-coagulant. 0.5 ml was dispensed into each of 12 centrifuge tubes.

3) 0, 5, 12.5, 20, 25 or 30 µl of each saponin solution was added to respective series of blood aliquots. The tubes were vortexed gently to mix, and incubated for 5 min at room temperature.

4) The tubes were centrifuged to pellet unlysed cells. 10 µl of each supernatant was diluted into 1 ml water. The optical density of each solution was read at 550 nm in a spectrophotometer.

The results of this determination are shown in Fig. 3. The overall trend observed for the saponinC-treated samples was comparable to previous results as shown above, e.g., the extent of hemolysis caused by 5 mg saponinC per ml of blood approaches the plateau of complete hemolysis. The plateau was approached at a lower concentration of saponinK.

The yield of total white blood cells and individual cell types was also determined for three levels of each saponin using methods as described above. The results are shown in Table 5:

## TABLE 5

| Sap-onin | mg ml Bld | Cells ml | Baso-phils | Eosin-ophils | Neutro-phils | Lympho-cytes | Mono-cytes |
|---|---|---|---|---|---|---|---|
| C | 1 | - | <0.5 | 3 | 67 | 25 | 5 |
| | 3 | $9.8 \times 10^5$ | <0.5 | 1.5 | 62 | 25 | 11.5 |
| | 5 | $2.5 \times 10^6$ | <0.5 | 1.5 | 46 | 32.5 | 20 |
| K | 1 | - | - | - | - | - | - |
| | 3 | $1.6 \times 10^6$ | <0.5 | 1.5 | 73.5 | 19 | 6 |
| | 5 | $3.8 \times 10^6$ | <0.5 | <0.5 | 21.5 | 71.5 | 7.5 |

For the cells obtained after treatment with 1 mg of either saponin per ml blood, the white blood cells could not be counted accurately because they were obscured by high red blood cell levels. This contamination may have also interfered with the Wright staining analysis of the saponinK sample since no white blood cells could be observed on the slide. The differential count ratios in these results show some variability. One disparity in particular is in the cell type ratios in the sample lysed with 5 mg saponinK per ml of blood. This condition, which may be due to susceptibility of neutrophils to lysis by this saponin prep, was observed several times in experiments using saponinK. For some purposes, e.g., preparing samples for HIV amplification, lysis of neutrophils might be considered an advantage, because neutrophils do not include target cells for HIV infection. For other purposes, saponinK might be judged unacceptable or best used at the lower level of 3 mg per ml blood.

Example 7: Dilution of Whole Blood Samples

When practical circumstances permit, the principles described herein can also be used to develop conditions in which the blood is diluted only a few fold (e.g. 1-5X) in the hemolysis steps. For example, the following procedure was used.

1) 0.5 ml samples of EDTA-treated whole blood were mixed with 0.5 ml of a 0.5% saponinK solution, and incubated at room temperature 5 min, and centrifuged to pellet white blood cells. The supernatant was aspirated and discarded.

2) The pellet was resuspended in 0.5 ml 1X $NH_4Cl$, incubated at room temperature 5 min, and centrifuged to pellet white blood cells. The supernatant was aspirated and discarded.

Example 8: Lysis of White Blood Cells

The white blood cells prepared by methods employing differential lysis of red blood cells can be lysed by any method known to the art, including by hydrolysis with a strong alkali. In other methods, for example, the cells can be lysed by osmotic shock or mild detergents and the intact nuclei recovered by centrifugation. The cells and nuclei can be completely lysed using more highly denaturing conditions, e.g., ionic detergents or chaotropic concentrations of a variety of solutes, and the released nucleic acids recovered by precipitation, ion exchange, hybridization selection etc. Other strategies, such as enzymatic digestion, for hydrolyzing non-target sample components can also be used.

1) White Blood Cell pellets were resuspended in 25 or 100 µl H₂O, and mixed with 25 or 100 µl 0.14 N KOH, respectively. The samples were vortexed to mix well, and heated at 95° for 30 min.

2) The pH of the resulting hydrolysate was adjusted to pH 8.0 ± 0.5 (typically) by adding 5 or 20 µl (respective to the volumes in step 1) of a solution comprising:

0.65 N HOAc
0.066 M Tris(-hydroxymethyl amino methane, base)
0.084 M Tris·HCl

The hydrolysate was mixed well, e.g., by vortexing.

Example 9: Target DNA Amplification in Lysates of White Blood Cells Prepared by Differential Lysis

The lysate resulting from these preparations can be added to a nucleic acid target amplification reaction, for example polymerase chain reaction (Mullis et al., supra) or by the method employing reverse transcriptase, RNase H and RNA polymerase activities as described by Kacian et al. supra. An example is described below.

1) 50 µl aliquots of a solution containing the following components were prepared and dispensed. The concentrations listed refer to the respective concentrations in the completed 100 µl reaction.

50 mM Tris·Cl (pH 8.0 at room
temperature)
17.5 mM MgCl₂
5 mM DTT
2 mM Spermidine
6.25 mM ea GTP & ATP
2.5 mM ea UTP & CTP
0.2 mM ea dATP, dGTP, dCTP, dTTP
0.3 µM ea primer "A" & primer "B"

2) Optionally, Deferoxamine mesylate at 0 - 1 mM final concentration may be included. Optionally, Zn(OAc)₂ at 0 - 0.1 mM final concentration may be included.

3) To this mixture, 40 µl of the hydrolyzed white blood cell solution prepared as described above was added. Optionally, a known amount of purified nucleic acid containing the target sequences of interest as a basis for comparing amplification performance supported by different conditions may be added. In this example, the lysates received 50 copies of HIV DNA purified from the BH10 clone.

4) The mixture was heated to 95°, maintained for 5 min, and cooled to 37°C.

5) 10 µl of a solution containing 600 U Moloney MuLV Reverse Transcriptase and 400 U T7 RNA polymerase, preferably in a buffered solution of composition sufficient to maintain enzyme stability during handling was added.

6) Samples were mixed briefly and incubated at 37°C for 1-2 hr.

7) The amount of amplification product formed as a copy of the intended target sequence may be determined using a specific hybridization procedure such as the hybridization protection assay (Arnold et al., supra). The following results are from the hybridization assay of Arnold, et al.,

TABLE 6

| Sample | HPA RLU |
| --- | --- |
| 55 µl Lysate of Saponin Prepared WBCs (50 BH10) | 454,305 |
| 220 µl Lysate of Saponin Prepared WBCs (50 BH10) | 389,723 |
| Amplification Negative Control | 1166 |
| HPA Negative Control | 849 |

## Claims

1.  A method of preparing nucleic acid from white blood cells for amplification and/or detection comprising the steps of:
    (a) contacting a white blood cell- and red blood cell-containing population of cells with a lysing agent under conditions in which red blood cells but not white blood cells are lysed, thereby leaving white blood cells intact;
    (b) purifying said intact white blood cells; and
    (c) contacting said intact white blood cells with a lysing agent able to lyse said white blood cells to release the nucleic acid sought to be amplified and/or detected.

2.  The method of claim 1 wherein at least about 95% of said red blood cells are lysed.

3.  The method of claim 1 wherein less than about 50% of said white blood cells are lysed.

4.  The method of claim 1 wherein at least about 80% of said red blood cells are lysed.

5.  The method of claim 1 wherein less than about 70% of said white blood cells are lysed.

6.  A method as claimed in any one of claims 1 to 5 wherein said population of cells is undiluted whole blood.

7.  A method as claimed in any one of claims 1 to 5 wherein said population of cells is whole blood diluted about 10-fold or less.

8.  The method of claim 7 wherein said population of cells is whole blood diluted about 5-fold or less.

9.  The method of claim 8 wherein said population of cells is whole blood diluted about 3-fold or less.

10. A method as claimed in any one of claims 6 to 9 wherein said blood is obtained from a human.

11. A method as claimed in any one of claims 1 to 10 wherein said lysing agent in step (a) is selected from saponins, detergents and haemolytic toxins.

12. The method of claim 11 wherein said lysing agent is a cationic detergent.

13. The method of claim 12 wherein said detergent is selected from cetrimide, lysolecithin, cetyl trimethylammonium bromide, dodecyl trimethylammonium bromide, tetradecyl trimethylammonium bromide and cetyl dimethylethylammonium bromide.

14. The method of claim 11 wherein said lysing agent is a toxin selected from lipases, macrolides and thiol-activated haemolysins.

15. A method as claimed in any one of claims 1 to 14 wherein in step (b) said intact white blood cells are purified by centrifugation.

16. A method as claimed in any one of claims 1 to 15 wherein in step (c) said intact white blood cells are lysed with alkali in an amount sufficient to cause said white blood cells to release said nucleic acid.

17. The method of claim 16 wherein said alkali is selected from KOH, $(CH_3)_3$ NOH, NaOH and LiOH.

18. A method as claimed in any one of claims 1 to 17 wherein said population of cells is present in a sample pretreated with an agent to physically remove at least some red blood cells.

19. A method as claimed in claim 18 wherein said pretreatment comprises contacting said sample with a carbohydrate polymer.

20. A method as claimed in claim 19 wherein said carbohydrate polymer is selected from dextran, hydroxyethyl starch and hydroxymethyl cellulose.

21. A method for amplifying nucleic acid from white blood cells comprising the steps of:
    (a) preparing said nucleic acid by a method as claimed in any one of claims 1 to 20; and

(b) amplifying said nucleic acid.

22. A method as claimed in claim 21 which further comprises contacting the white blood cell lysate obtained in step (a) with a complexing agent, which complexes with ferric ions but does not complex with zinc, manganese or magnesium ions, prior to amplifying the nucleic acid.

23. A kit for preparation of nucleic acid for amplification according to any one of claims 1 to 20 comprising:
   a) a first vessel containing a lysing agent capable of differentially lysing red blood cells and white blood cells; and
   b) a second vessel containing a lysing agent capable of lysing white blood cells containing said nucleic acid.

24. The kit of claim 23 further comprising a device for the collection and storage of whole blood.

25. A kit as claimed in claim 23 or claim 24 further comprising a vessel comprising a probe for screening said nucleic acid for a viral nucleic acid sequence.

26. The kit of claim 25 wherein said viral nucleic acid sequence is a nucleic acid sequence of a human immunodeficiency virus.

Hemolysis by Low Concentrations of
Cytolytic Agents in Whole Blood

## Identification of Cytolytic Agent Level
## Sufficient for Complete Hemolysis

Comparison of Hemolytic Effectiveness
of Two Saponin Preparations